# EUROPEAN PATENT SPECIFICATION

(11) **EP 3 391 967 B1**
(45) Date of publication and mention of the grant of the patent: **01.04.2020**
(21) Application number: 17191599.4
(22) Date of filing: 18.09.2017
(51) Int. Cl.: B01L 3/00, B81B 7/00, A61B 10/00

(54) **DETECTION APPARATUS AND INLET STRUCTURE THEREOF**
DETEKTIONSVORRICHTUNG UND EINLASSSTRUKTUR DAVON
APPAREIL DE DÉTECTION ET SA STRUCTURE D'ENTRÉE

(30) Priority: 19.04.2017 CN 201710256374
(43) Date of publication of application: 24.10.2018
(73) Proprietor: Skyla Corporation Hsinchu Science Park Branch, Hsinchu City 30078 (TW)
(72) Inventor: WEI, Chia-Chun, 11492 Neihu, Taipei (TW); CHEN, Hung-Wei, 11492 Neihu, Taipei (TW)
(74) Representative: 2K Patentanwälte Blasberg Kewitz & Reichel

(56) References cited:
- WO-A1-03/103835
- US-A1- 2004 241 051
- US-A1- 2005 169 778
- US-A1- 2009 045 058
- US-A1- 2011 011 781
- US-A1- 2012 156 765
- US-A1- 2014 134 595

## Description

### BACKGROUND OF THE INVENTION

### 1. Field of the Invention

The invention is directed to a detection apparatus and an inlet structure thereof and more particularly, to an inlet structure capable of destroying a surface tension of a fluid and a detection apparatus applying the inlet structure.

### 2. Description of Related Art

To satisfy demands of emphasis on preventive medicine, early diagnosis and early treatment in medical treatment, demands on automated testing environment, point of care (POC) or near patient testing and molecular diagnosis have increased. Currently, most of the blood samples are still separated in the centrifuge, and fluids to be tested after the separation are further taken therefrom to a detection apparatus for testing. The detection apparatus may be, for example, a detector instrument or a rapid-screening cassette.
US2014/134595 A1 discloses a device and a method for filtering a liquid sample, wherein first capillary-driven filtration occurs and, after initial filling, pressure-operated filtration is performed by applying a vacuum or a positive pressure.
US2004241051 A1 relates to devices for efficient transport, transfer and movement of fluids. In particular, the invention provides fluidic micro-structures for controlled transport and movement of liquids in devices for analytical and other purposes. Devices of the invention include one or more features that can enhance performance of the fluid transfer, referred to as a pre-shooter stop, a butterfly structure, a cascade structure, a waste chamber inlet, a capillary driven sample inlet chamber, a capillary stop structure, a bifurcation flow-through structure, and a hydrophobic vent.
US2011/011781 A1 relates to an apparatus for separating blood, more particularly an apparatus for absorbing blood and separating blood components, e.g. blood plasma, as a sample liquid. Said apparatus comprises a feeding device for absorbing the blood, a device for separating blood components as a sample liquid, a duct which preferably absorbs the sample liquid exclusively by means of capillary forces, and a device for filling the duct with sample liquid in an inlet or feeding zone of the duct. The separating device, in particular a membrane, is curved, especially convexly shaped, and the apex of said curved, especially convex shape projects into the filling device.
US2005/169778 A1 relates to a microstructure arrangement for the bubble-free filling with a liquid of at least one system for draining off liquids. The arrangement has an inlet for the arrangement to be connected to a system for the supply of liquids and at least one outlet for the arrangement to be connected to the at least one liquid-discharging system. The arrangement has a transition region, through which the liquid can be transported from the inlet to the at least one outlet. At a start of the transition region, at least one first microstructure element for producing a point with increased capillary force is provided, in order to achieve gap-free wetting of the areas bounding this point with increased capillary force, in particular side walls, a cover and/or a bottom.
US2009/045058 A1 relates to a microchip used to implement a separation operation of an analysis sample without contamination or spillage, composed of a substrate and a cover, the substrate being provided with a trench-shaped channel in its upper surface and substrate reservoirs that are linked with this channel, and the cover hermetically sealing the upper surface of the channel, attachable to or detachable from the substrate, and provided with through-holes formed at positions corresponding to the substrate reservoirs, cover reservoirs that are formed on the inner sides of the through-holes for holding liquid that is introduced when the cover is in the state of hermetically sealing the upper surface of the channel, and partitions that are formed on the bottom surfaces of the cover reservoirs. The areas of the openings of the partitions are smaller than the areas of the openings of the cover reservoirs that are over the partitions.
WO03103835 A1 relates to a micro fluidic system comprising a substrate, and, provided on said substrate, at least one flow path interconnecting with functional means in which liquid samples can be treated by desired procedures. The flow paths are laid out to form a pattern for the transport of liquid samples to and from said functional means. These flow paths comprise a plurality of micro posts protruding upwards from said substrate, the spacing between the micro posts being small enough to induce a capillary action in a liquid sample applied anywhere within any of said flow paths, so as to force said liquid to move from where said liquid sample was applied.
US2012156765 A1 relates to a fluid filtering device comprising a filter for filtering a fluid and a substrate comprising a collecting structure for collecting the filtered fluid from the filter. An adhesive holds the filter in place with respect to the collecting structure. The filter, the substrate with the collecting structure and the adhesive are arranged such that the collecting structure contacts the filter and the filter is attached to the substrate by the adhesive. The collecting structure is very close to the filter and the adhesive ensures that the collecting structure remains very close to the filter. The filtered fluid can therefore be collected very efficiently and very fast. The fluid filtering device is preferentially used for filtering blood in a biosensing device.

In a conventional detection apparatus, when a fluid is dropped into an inlet of the detection apparatus, the behavior of the fluid is influenced by the surface tension easily and has difficulty of being guided into the inlet. Thus, an absorbent material has to be additionally used to guide and absorb the fluid to allow the fluid to pass through the inlet and enter into the detection apparatus. In this way, production cost of the detection apparatus is increased.

### SUMMARY OF THE INVENTION

The invention is as defined in the independent claim 1. The dependent claims are directed to beneficial embodiments.

The invention provides an inlet structure capable of destroying a surface tension of a fluid to cause the fluid to flow into a microchannel by a capillary action according to the claims.

The invention provides a detection apparatus capable of allowing a fluid to flow into an inlet structure and a microchannel without the use of any absorbent material, which benefits in cost reduction.

An inlet structure of the invention is connected to a microchannel. The inlet structure includes an inlet portion and at least one micro structure. The inlet portion has an inner surface. The micro structure is disposed in the inlet portion and connected with the inner surface. When a fluid is injected into inlet portion, the micro structure destroys the surface tension of the fluid to cause the fluid to flow into the microchannel by a capillary action.

A detection apparatus of the invention includes at least one inlet structure and at least one microchannel. The inlet structure includes an inlet portion and at least one micro structure. The inlet portion has an inner surface. The micro structure is disposed in the inlet portion and connected to the inner surface. The microchannel is connected to the inlet structure. When a fluid is injected into the inlet portion, the micro structure destroys a surface tension of the fluid to cause the fluid to flow into the microchannel by a capillary action. Furthermore, the micro structure is a convex-shaped micro structure protruding from the inner surface of the inlet portion, or the micro structure is a concaved-shaped micro structure buried into the inner surface of the inlet portion. The inlet portion further has an inclined surface and a bottom surface. The inner surface is connected between the inclined surface and the bottom surface, and the inner surface is perpendicularly or inclinedly connected to the bottom surface.

In an embodiment of the invention, when the number of the micro structure is plural, the plurality of micro structures are arranged and spaced equidistantly.

In an embodiment of the invention, a side of the micro structure is located at a junction between the inclined surface and the inner surface.

In an embodiment of the invention, a vertical spacing is between a lower surface of the micro structure and the bottom surface of the inlet portion.

In an embodiment of the invention, a lower surface of the micro structure is aligned with the bottom surface of the inlet portion.

In an embodiment of the invention, a top-viewed shape of the inlet portion is circular, and a diameter of the microchannel is less than or equal to a diameter of the inlet portion.

In an embodiment of the invention, a top-viewed shape of the micro structure is polygonal, arcuate or irregular-shaped.

Based on the above, in the inlet structure of the invention, because at least one micro structure is disposed therein, and when the fluid is injected into the inlet portion of the inlet structure, the micro structure can destroy the surface tension of the fluid to cause the fluid to flow into the microchannel by the capillary action. In this way, the inlet structure can guide the fluid to flow inward without the use of any absorbent material. Moreover, the detection apparatus of the invention includes the aforementioned inlet structure which can achieve demands for cost reduction as the use of the absorbent material is not required.

Several exemplary embodiments accompanied with figures are described in detail below to further describe the invention in details.

### BRIEF DESCRIPTION OF THE DRAWINGS

The accompanying drawings are included to provide a further understanding of the present invention, and are incorporated in and constitute a part of this specification. The drawings illustrate embodiments of the present invention and, together with the description, serve to explain the principles of the present invention.
FIG. 1 is a partial top view of a detection apparatus according to an embodiment of the invention.
FIG. 2 is a partial perspective cross-sectional view of the detection apparatus depicted in FIG. 1.
FIG. 3 is a perspective cross-sectional view of an inlet structure according to another embodiment of the invention.
FIG. 4 is a top view of an inlet structure according to yet another embodiment of the invention.

### DESCRIPTION OF THE EMBODIMENTS

Reference will now be made in detail to the present preferred embodiments of the invention, examples of which are illustrated in the accompanying drawings. Wherever possible, the same reference numbers are used in the drawings and the description to refer to the same or like parts.

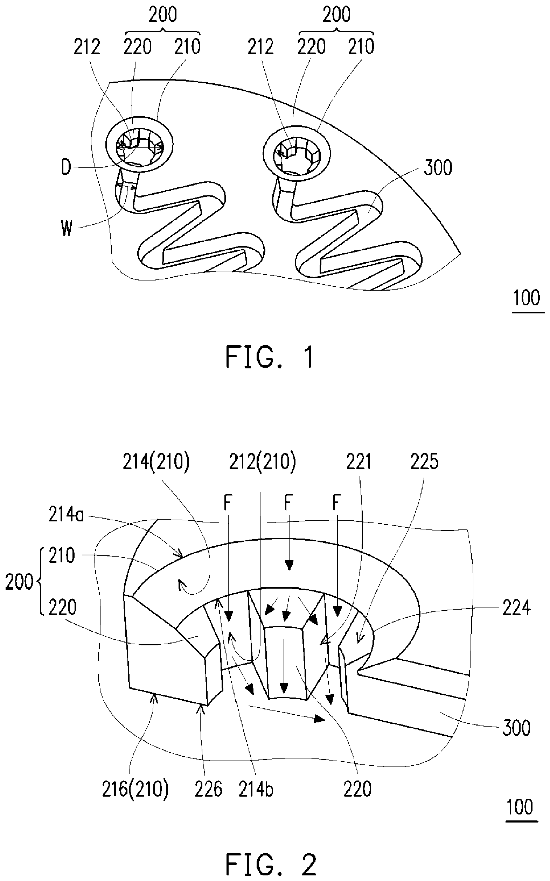

FIG. 1 is a partial top view of a detection apparatus according to an embodiment of the invention. FIG. 2 is a partial perspective cross-sectional view of the detection apparatus depicted in FIG. 1. Referring to both FIG. 1 and FIG. 2, a detection apparatus 100 of the present embodiment includes at least one inlet structure 200 (which only two are schematically illustrated in FIG. 1) and at least one microchannel 300 (which only two are schematically illustrated in FIG. 1). Each of the inlet structures 200 is adapted to be respectively connected to each of the microchannels 300.

To be specific, each of the inlet structures 200 of the detection apparatus 100 of the present embodiment includes an inlet portion 210 and at least one micro structures 220 (which five are schematically illustrated in FIG. 1). The inlet portion 210 has an inner surface 212, and the micro structure 220 is disposed in the inlet portion 210 and connected to the inner surface 212. Particularly, when a fluid F is injected into the inlet portion 210, the micro structure 220 may destroy a surface tension of the fluid F to cause the fluid F to flow into the microchannel 300 by a capillary action.

To be more specific, referring again to FIG. 2, the inlet portion 210 of the present embodiment further has an inclined surface 214, which is inclined downward, and a bottom surface 216. The inner surface 212 is connected between the inclined surface 214 and the bottom surface 216, and the inner surface 212 is perpendicularly connected to the bottom surface 216. The design of the inclined surface 214 is capable of guiding the fluid F to enter into the inner surface 212. However, the inner surface 212 may have an inclined angle (which is also referred to as a draft angle) with respect to the bottom surface 216, but the invention is not limited thereto. As illustrated in FIG. 2, a top-viewed shape of the inlet portion 210 of the present embodiment is embodied as a circle. A diameter of the inlet portion 210 is gradually reduced from a side 214a of the inclined surface 214 which is farthest away from the inner surface 212 toward another side 214b of the inclined surface 214 which is closest to the inner surface 212. In other words, the diameter of the inlet portion 210 of the present embodiment is not a fixed value, but the invention is not limited thereto.

Additionally, in the present embodiment, the plurality of micro structures 220 are arranged and spaced equidistantly, wherein the micro structures 220 are convex-shaped micro structures, namely, the micro structures 220 are disposed on the inner surface 212 in a manner of protruding therefrom. As illustrated in FIG. 2, a side 224 of the micro structure 220 is located at a junction between the inclined surface 214 and the inner surface 212. Nevertheless, the side 224 may be disposed lower than the junction between the inclined surface 214 and the inner surface 212. An upper surface 225 of each micro structure 220 is an inclined surface which is inclined downward. In FIG. 2, an inclined angle of the upper surface 225 is greater than an inclined angle of the inclined surface 214, but the invention is not limited thereto. Each micro structure 220 has three side surfaces 221 (only one of the side surfaces 221 is illustrated in FIG. 2). Two of the side surfaces 221 are connected to the inner surface 212, and the other side surface 221 is connected between the two side surfaces 221. The side surfaces 221 are perpendicularly disposed on the bottom surface 216. However, the side surfaces 221 may be disposed at an inclined angle (which is also referred to as a draft angle) with respect to the bottom surface 216, which is not limited by the invention. A lower surface 226 of each micro structure 220 is aligned with the bottom surface 216 of the inlet portion 210. In other words, a height of the micro structure 220 is substantially the same as a depth of the inner surface 212, but the invention is not limited thereto. In this case, a top-viewed shape of each micro structure 220 is, for example, polygonal, arcuate or irregular-shaped, but the invention is not limited thereto.

As the inlet structure 200 of the present embodiment are disposed with the micro structures 220, when the fluid F is injected into the inlet portion 210 of the inlet structure 200, the micro structures 220 are capable of destroying the surface tension of the fluid F to cause the fluid F to flow into the microchannel 300 connected to the inlet structure 200 by the capillary action. In this way, the inlet structure 200 can guide the fluid F to flow into the inlet structure 200 and the microchannel 300, without the use of any absorbent material. In other words, the detection apparatus 100 of the present embodiment can satisfy the demand for cost reduction.

In addition, the microchannel 300 of the present embodiment are connected to the inlet structure 200, thereby guiding the fluid F to flow into a reaction tank (which is not shown) of the detection apparatus 100 for subsequent analysis. Preferably, a diameter W of the microchannel 300 is less than or equal to a diameter D (which refers to an inner diameter of the inner surface 212 in this case) of the inlet portion 210 to cause the fluid F to flow into the microchannel 300 more easily by the capillary action.

It should be noted that the reference numerals and a part of the contents in the aforementioned embodiment are used in the following embodiments, in which identical reference numerals are adopted to represent identical or similar components, and repeated descriptions of the same technical contents are omitted. For detailed descriptions of the omitted parts, a reference can be found in the aforementioned embodiment, and repeated descriptions thereof are omitted in the following embodiments.

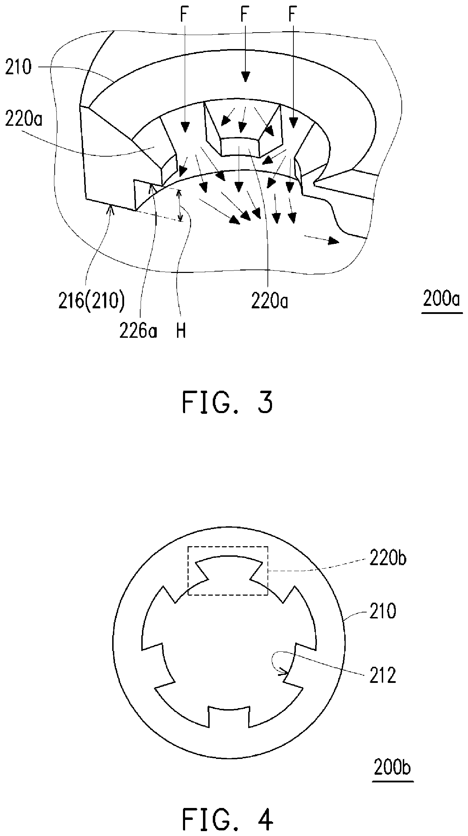

FIG. 3 is a perspective cross-sectional view of an inlet structure according to another embodiment of the invention. Referring to both FIG. 2 and FIG. 3, an inlet structure 200a of the present embodiment is substantially similar to the inlet structure 200 illustrated in FIG. 2, and the difference between the two lies in that there is an vertical spacing H between a lower surface 226a of each micro structure 220a and the bottom surface 216 of the inlet portion 210 in the present embodiment. In other words, the lower surface 226a of each micro structure 220a and the bottom surface 216 of the inlet portion 210 are not on the same plane. When the fluid F is injected into the inlet portion 210 of the inlet structure 200a, the micro structure 220a is capable of destroying the surface tension of the fluid F, and the vertical spacing H between the lower surface 226a of the micro structure 220a and the bottom surface 216 of the inlet portion 210 produces the capillary force more favorably, such that the fluid F can flow into the microchannel 300 connected to the inlet structure 200a more easily by the capillary action.

FIG. 4 is a top view of an inlet structure according to yet another embodiment of the invention. Referring to both FIG. 2 and FIG. 4, an inlet structure 200b of the present embodiment is substantially similar to the inlet structure 200 illustrated in FIG. 2, and the difference between the two lies in that micro structures 220b of the inlet structure 200b of the present embodiment are a plurality of concaved-shaped micro structures, where the micro structures 220b are buried into the inner surface 212 of the inlet portion 210. With the design of angles and sizes of the concavities, the micro structures 220b are capable of destroying the surface tension of the fluid F to cause the fluid F to easily flow into the microchannel 300.

It is to be mentioned that the structural shapes of the micro structures 220, 220a and 220b are not limited by the invention, and it falls within the scope of protection by the invention as long as a non-smooth surface can be defined by the micro structures 220, 220a and 220b and the inner surface 212 of the inlet portion 210. Based on the above design principle, the top-viewed shape of the micro structures 220, 220a and 220b may not only be polygonal, but also arcuate or other irregular-shapes in other embodiments that are not shown.

In view of the foregoing, the detection apparatus of the invention has the inlet structure, and the inlet structure is disposed with the micro structures. Thus, when the fluid is injected into the inlet portion of the inlet structure, the micro structures can destroy the surface tension of the fluid to cause the fluid to flow into the microchannel connected to the inlet structure by the capillary action. In this way, the inlet structure of the invention can guide the fluid to flow into the microchannel without the use of any absorbent material. On the other hand, as the detection apparatus of the invention does not need any absorbent material, the detection apparatus of the invention can satisfy the demand for cost reduction.

## Claims

1. A detection apparatus (100), comprising:
an inlet structure (200, 200a, 200b), comprising:
an inlet portion (210), having an inner surface (212); and
at least one micro structure (220, 220a, 220b), disposed in the inlet portion (210) and connected with the inner surface (212), and
a microchannel (300), connected to the inlet structure (200, 200a, 200b),
wherein when a fluid (F) is injected into the inlet portion (210), the micro structure (220, 220a, 220b) destroys a surface tension of the fluid (F) to cause the fluid (F) to flow into the microchannel (300) by a capillary action,
**characterized in that** the inlet portion (210) further has an inclined surface (214) and a bottom surface (216), the inner surface (212) is connected between the inclined surface (214) and the bottom surface (216), and the inner surface (212) is perpendicularly or inclinedly connected to the bottom surface (216);
and wherein the micro structure (220, 220a) is a convex-shaped micro structure (220, 220a) protruding from the inner surface (212) of the inlet portion (210) or the micro structure (220b) is a concaved-shaped micro structure (220b) buried into the inner surface (212) of the inlet portion (210).

2. The detection apparatus (100) as recited in claim 1, wherein when the number of the micro structure (220, 220a, 220b) is plural, the plurality of micro structures (220, 220a, 220b) are arranged and spaced equidistantly.

3. The detection apparatus (100) as recited in any of the claims 1 to 2, wherein a side (224) of the micro structure (220, 220a) is located at a junction between the inclined surface (214) and the inner surface (212).

4. The detection apparatus (100) as recited in claim 1 or 3, wherein a vertical spacing (H) is between a lower surface (226a) of the micro structure (220a) and the bottom surface (216) of the inlet portion (210).

5. The detection apparatus (100) as recited in claim 1 or 3, wherein a lower surface (226) of the micro structure (220) is aligned with the bottom surface (216) of the inlet portion (210).

6. The detection apparatus (100) as recited in any of the claims 1 to 5, wherein a top-viewed shape of the inlet portion (210) is circular, and a diameter (W) of the microchannel (300) is less than or equal to a diameter (D) of the inlet portion (210).

7. The detection apparatus (100) as recited in any of the claims 1 to 6, wherein a top-viewed shape of the micro structure (220, 220a, 220b) is polygonal, arcuate or irregular-shaped.

## Patentansprüche

1. Ein Detektionsapparat (100), bestehend aus:
eine Einlassstruktur (200, 200a, 200b), bestehend aus:
einen Einlassabschnitt (210) mit einer Innenfläche (212); und
mindestens eine Mikrostruktur (220, 220a, 220b), die im Einlassabschnitt (210) angeordnet und mit der Innenfläche (212) verbunden ist, und
einen Mikrokanal (300), der mit der Einlassstruktur (200, 200a, 200b) verbunden ist,
wobei, wenn ein Fluid (F) in den Einlassabschnitt (210) injiziert wird, die Mikrostruktur (220, 220a, 220b) eine Oberflächenspannung des Fluids (F) zerstört, um zu bewirken, dass das Fluid (F) durch eine Kapillarwirkung in den Mikrokanal (300) fließt,
**dadurch gekennzeichnet, dass** der Einlassabschnitt (210) ferner eine geneigte Fläche (214) und eine Bodenfläche (216) aufweist, die Innenfläche (212) zwischen der geneigten Fläche (214) und der Bodenfläche (216) verbunden ist und die Innenfläche (212) senkrecht oder geneigt mit der Bodenfläche (216) verbunden ist;
und wobei die Mikrostruktur (220, 220a) eine konvex geformte Mikrostruktur (220, 220a) ist, die von der Innenfläche (212) des Einlassabschnitts (210) vorsteht, oder die Mikrostruktur (220b) eine konkav geformte Mikrostruktur (220b) ist, die in die Innenfläche (212) des Einlassabschnitts (210) eingebettet ist.

2. Die Detektionsvorrichtung (100) nach Anspruch 1, wobei, wenn die Anzahl der Mikrostrukturen (220, 220a, 220b) eine Mehrzahl ist, die Mehrzahl der Mikrostrukturen (220, 220a, 220b) in gleichem Abstand angeordnet und beabstandet sind.

3. Die Detektionsvorrichtung (100) nach einem der Ansprüche 1 bis 2, wobei eine Seite (224) der Mikrostruktur (220, 220a) an einer Verbindung zwischen der geneigten Oberfläche (214) und der Innenfläche (212) angeordnet ist.

4. Die Detektionsvorrichtung (100) nach Anspruch 1 oder 3, wobei ein vertikaler Abstand (H) zwischen einer unteren Oberfläche (226a) der Mikrostruktur (220a) und der unteren Oberfläche (216) des Einlassabschnitts (210) besteht.

5. Die Detektionsvorrichtung (100) nach Anspruch 1 oder 3, wobei eine untere Oberfläche (226) der Mikrostruktur (220) mit der unteren Oberfläche (216) des Einlassabschnitts (210) ausgerichtet ist.

6. Die Detektionsvorrichtung (100) nach einem der Ansprüche 1 bis 5, wobei eine von oben gesehene Form des Einlassabschnitts (210) kreisförmig ist und ein Durchmesser (W) des Mikrokanals (300) kleiner oder gleich einem Durchmesser (D) des Einlassabschnitts (210) ist.

7. Die Detektionsvorrichtung (100) nach einem der Ansprüche 1 bis 6, wobei die Mikrostruktur (220, 220a, 220b) in der Draufsicht polygonal, bogenförmig oder unregelmäßig geformt ist.

## Revendications

1. Un appareil de détection (100), comprenant :
une structure d'entrée (200, 200a, 200b), comportant :
une partie d'entrée (210), ayant une surface intérieure (212); et
au moins une microstructure (220, 220a, 220b), disposée dans la partie d'entrée (210) et raccordée à la surface intérieure (212), et
un microcanal (300), raccordé à la structure d'entrée (200, 200a, 200b),
dans lequel lorsqu'un fluide (F) est injecté dans la partie d'entrée (210), la microstructure (220, 220a, 220b) détruit une tension superficielle du fluide (F) pour amener le fluide (F) à s'écouler dans le microcanal (300) par une action capillaire,
**caractérisé en ce que** la partie d'entrée (210) a en outre une surface inclinée (214) et une surface inférieure (216), la surface intérieure (212) étant connectée entre la surface inclinée (214) et la surface inférieure (216), et la surface intérieure (212) est reliée perpendiculairement ou inclinée à la surface inférieure (216) ;
et dans lequel la microstructure (220, 220a) est une microstructure (220, 220a) de forme convexe faisant saillie par rapport à la surface interne (212) de la partie d'entrée (210) ou la microstructure (220b) est une microstructure (220b) de forme concave enfouie dans la surface intérieure (212) de la partie d'entrée (210).

2. L'appareil de détection (100) tel que défini dans la revendication 1, dans lequel lorsque la microstructure (220, 220a, 220b) est multiple, les multiples microstructures (220, 220a, 220b) sont disposées et espacées de manière équidistante.

3. L'appareil de détection (100) tel que défini dans l'une quelconque des revendications 1 à 2, dans lequel un côté (224) de la microstructure (220, 220a) est située à une jonction entre la surface inclinée (214) et la surface interne (212).

4. L'appareil de détection (100) tel que défini dans la revendication 1 ou 3, dans lequel un espacement vertical (H) est entre une surface inférieure (226a) de la microstructure (220a) et la surface inférieure (216) de la partie d'entrée (210).

5. L'appareil de détection (100) tel que défini dans la revendication 1 ou 3, dans lequel une surface inférieure (226) de la microstructure (220) est alignée avec la surface inférieure (216) de la partie d'entrée (210).

6. L'appareil de détection (100) tel que défini dans l'une quelconque des revendications 1 à 5, dans lequel une forme vue de dessus de la partie d'entrée (210) est circulaire, et un diamètre (W) du microcanal (300) est inférieur ou égal à un diamètre (D) de la partie d'entrée (210).

7. L'appareil de détection (100) tel que défini dans l'une quelconque des revendications 1 à 6, dans lequel une forme vue de dessus de la microstructure (220, 220a, 220b) est polygonale, arquée ou de forme irrégulière.
